# EUROPEAN PATENT APPLICATION

(11) **EP 4 520 351 A1**
(43) Date of publication of application: **12.03.2025**
(21) Application number: 23799516.2
(22) Date of filing: 02.05.2023
(51) Int. Cl.: A61L 2/10

(54) **FLUID STERILIZATION DEVICE**

(30) Priority: 02.05.2022 JP 2022075966
(71) Applicant: Stanley Electric Co., Ltd., Meguro-ku Tokyo 153-8636 (JP)
(72) Inventor: TANAKA, Hideaki, Tokyo 153-8636 (JP); KATO, Hiroyuki, Tokyo 153-8636 (JP); SHINNO, Kazuhisa, Tokyo 153-8636 (JP)
(74) Representative: Klang, Alexander H.
(86) International application number: PCT/JP2023/017178
(87) International publication number: WO 2023/214587

(57) **Abstract**

An object is to provide a fluid sterilization device capable of achieving a compact device even if a heat dissipation structure such as a substrate, on which electronic components other than the light emitting element are mounted, or a heat sink is provided.

A fluid sterilization device includes a cylindrical flow path pipe including a side peripheral wall and a flow path formed inside the side peripheral wall; a light source unit including a light emitting element, an electronic component other than the light emitting element; and a substrate on which the light emitting element and the electronic component are mounted and that extends in a longitudinal direction of the flow path pipe; and a heat sink in thermal contact with an outer surface of the substrate. The flow path pipe further includes a substrate placement table portion for placing the substrate, and the substrate placement table portion includes a first through hole, for accommodating the light emitting element, that penetrates through to the flow path, and a first recess for accommodating the electronic component.

## Description

### Technical Field

The present invention relates to a fluid sterilization device.

### Background Art

Various products have been provided for sterilizing fluids (e.g., liquid for beverages or raw water for food, or various types of cooling water and washing water for use in factories) by irradiation with ultraviolet light. Among inventions related to these products, a fluid sterilization device provided in a fluid flow path is disclosed in, for example, Patent Literature 1 below.

The fluid sterilizing apparatus disclosed in Patent Literature 1 includes a flow path pipe including a flow path through which a fluid to be sterilized flows, an ultraviolet ray generating device installed outside the flow path pipe, the ultraviolet ray generating device being configured to irradiate the above-described fluid with ultraviolet rays, and a seal member for closing a gap between the flow path pipe and the ultraviolet ray generating device. Here, the ultraviolet light generating device includes a housing, which is fixed to the flow path pipe, and a light emitting element (light source), which is installed in the housing and is configured to emit ultraviolet light. Furthermore, the housing includes a convex portion that is located around the light emitting element and that protrudes toward the inside of the flow path pipe, so as to be located between the light source and the seal member. Furthermore, the flow path pipe includes a side wall portion that is located between the flow path and the seal member and that protrudes toward the convex portion. Furthermore, the convex portion and the side wall portion are formed of a material that shields ultraviolet rays. Furthermore, the seal member is separated, by the convex portion and the side wall portion, away from the flow path and also the space where the light emitting element is disposed.

### Citation List

### Patent Literature

Patent Literature 1**:** Japanese Patent Application Laid-Open No. 2021-122516

### Summary of Invention

### Technical Problem

When, for example, LED (Light emitting diode) is used as the light emitting element as in the fluid sterilization device of Patent Literature 1, various electronic components for driving the LED need to be provided via a predetermined (circuit) substrate. However, if the LED and the substrate on which the electronic components and the like are mounted are provided without any contrivance in the fluid sterilization device of Patent Literature 1**,** there is a possibility that the size of the device may increase.

Furthermore, it is preferable to provide a heat sink that dissipates, to the outside of the flow path pipe, heat generated when the light emitting element (LED or the like) is driven, from the viewpoint of ensuring stable driving of the light emitting element, the viewpoint of preventing a rise in temperature of the fluid flowing in the flow path pipe, and the like. However, if the fluid sterilization device of Patent Literature 1 is provided with such a heat dissipation structure including the heat sink as it is without any contrivance, the size of the device may increase as in the case of installing the above-described substrate.

In view of the above-mentioned problems, an object of the present invention is to provide a fluid sterilization device capable of achieving device miniaturization even if the device is equipped with a substrate, on which electronic components other than a light emitting element are mounted, or a heat dissipation structure such as a heat sink.

### Solution to Problem

In order to solve the above-described problems, a fluid sterilization device according to the present invention includes:
a cylindrical flow path pipe including a side peripheral wall and a flow path formed inside the side peripheral wall;
a light source unit including a light emitting element, an electronic component other than the light emitting element; and a substrate on which the light emitting element and the electronic component are mounted and that extends in a longitudinal direction of the flow path pipe; and
a heat sink in thermal contact with an outer surface of the substrate, wherein:
   the flow path pipe further includes a substrate placement table portion for placing the substrate; and
   the substrate placement table portion includes
      a first through hole, for accommodating the light emitting element, that penetrates through to the flow path, and
      a first recess for accommodating the electronic component.

In this aspect of the present invention, the substrate (and the heat sink in thermal contact with the outer surface of the substrate) on which the light emitting element and the electronic component other than the light emitting element are mounted is placed on the substrate placement table portion of the flow path pipe. Furthermore, when the substrate is placed on the substrate placement table portion, the light emitting element faces the first through hole that penetrates from the substrate placement table portion to the flow path of the flow path pipe, and the electronic component other than the light emitting element is accommodated in the first recess of the substrate placement table portion. Thus, according to this aspect of the present invention, when the substrate and the substrate placement table portion overlap, the light emitting element and the electronic components mounted on the substrate, and the substrate placement table portion (the side peripheral wall of the flow path pipe) can be prevented from physically obstructing each other. As a result, the device can be miniaturized.

Furthermore, the fluid sterilization device according to the present invention preferably further includes a metal screw or pin for fixing the heat sink and the substrate to the substrate placement table portion, and
the fluid sterilization device is preferably configured such that:
a second through hole is provided in the heat sink;
a third through hole is provided at a position overlapping the second through hole in the substrate;
a second recess is provided at a position overlapping the second through hole and the third through hole in the substrate placement table portion; and
the screw or pin is inserted into the second through hole, the third through hole, and the second recess.

According to this aspect of the present invention, the heat sink and the substrate are fixed to the substrate placement table portion via the metal screw or pin, and thus, it is possible to effectively prevent the heat sink and the substrate from being detached from the substrate placement table portion. In addition to this, it is possible to appropriately dissipate, via the metal screw or pin, heat generated in the substrate when the light emitting element is driven (when a current is applied thereto).

Furthermore, in the fluid sterilization device according to the present invention,
it is preferable that the metal screw include a metal fixing screw part to be inserted into the second through hole and the third through hole, and a metal insert screw part disposed in the second recess and screwed with the fixing screw part.

According to this aspect of the present invention, the metal screw is configured to include the metal fixing screw part to be inserted into the second through hole of the heat sink and the third through hole of the substrate, and the metal insert screw part disposed in the second recess of the substrate placement table portion. Thus, it is possible to more firmly fix the heat sink and the substrate to the substrate placement table portion. In addition, according to this aspect of the present invention, heat dissipation can be enhanced.

Furthermore, the fluid sterilization device according to the present invention preferably further includes a housing, and
is preferably configured such that:
the housing includes a hollow base portion having an opening at least on the top side, and a lid portion configured to cover at least a part of the opening;
the flow path pipe is accommodated in the base portion; and
an outer surface of the heat sink faces the opening of the base portion to be in contact with the lid portion.

According to this aspect of the present invention, the flow path pipe is accommodated in the base portion of the housing and the outer surface of the heat sink is in contact with the lid portion of the housing, whereby detachment of the heat sink and the substrate can be prevented. In addition, according to this aspect of the present invention, the use of a fan or a large heat sink provided with fins is not necessary, allowing for device miniaturization.

Furthermore, the fluid sterilization device according to the present invention is preferably configured such that the flow path pipe includes a main pipe portion and an inlet pipe portion having an inner diameter smaller than an inner diameter of the main pipe portion.

According to this aspect of the present invention, the main pipe portion of the flow path pipe with a larger inner diameter than the inlet pipe portion allows the fluid flow rate through the flow path to slow down. As a result, the amount of ultraviolet light emitted from the light emitting element with respect to the fluid flowing through the main tube portion can be increased. As a result, the sterilization function can be further improved.

Furthermore, in the fluid sterilization device according to the present invention,
the substrate and the outer surface of the flow path pipe are preferably in thermal contact with each other.

According to this aspect of the present invention, heat generated by the light emitting element or the electronic component mounted on the substrate can be conducted to the flow path pipe. Thus, the heat dissipation property can be enhanced.

### Advantageous Effects of Invention

According to the present invention, it is possible to provide a fluid sterilization device capable of achieving miniaturization of the device even if a substrate, on which electronic components other than a light emitting element are mounted, or a heat dissipation structure such as a heat sink, is provided.

### Brief Description of Drawings

[Fig. 1] Fig. 1 is a vertical cross-sectional view of a fluid sterilization device according to an embodiment of the present invention.
[Fig. 2] Fig. 2 is an exploded perspective view of the fluid sterilization device according to the present embodiment.
[Fig. 3] Fig. 3 is an exploded perspective view of the fluid sterilization device according to the present embodiment.
[Fig. 4] Fig. 4 is an exploded perspective view of a fluid sterilization device according to a modification. Description of Embodiments

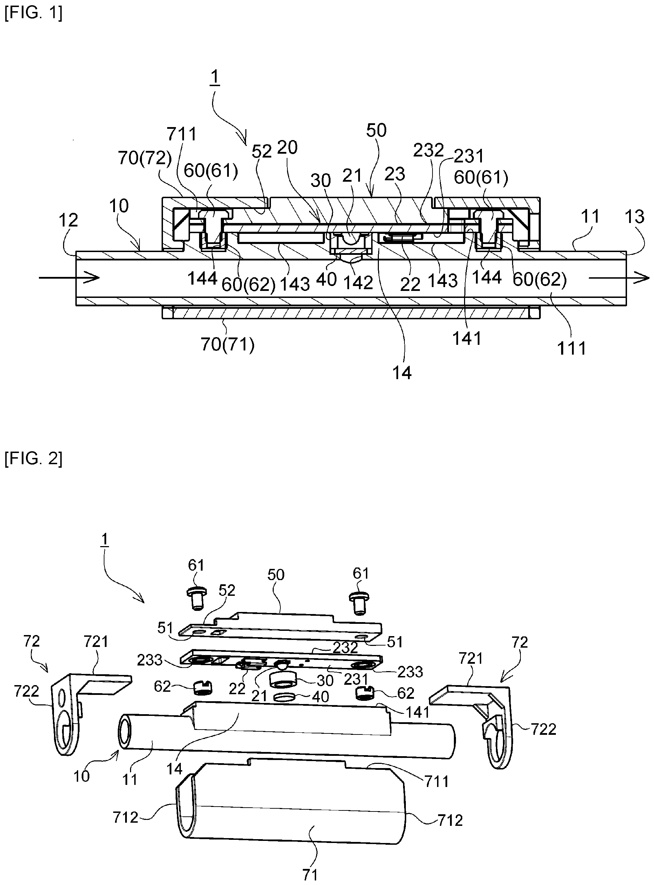

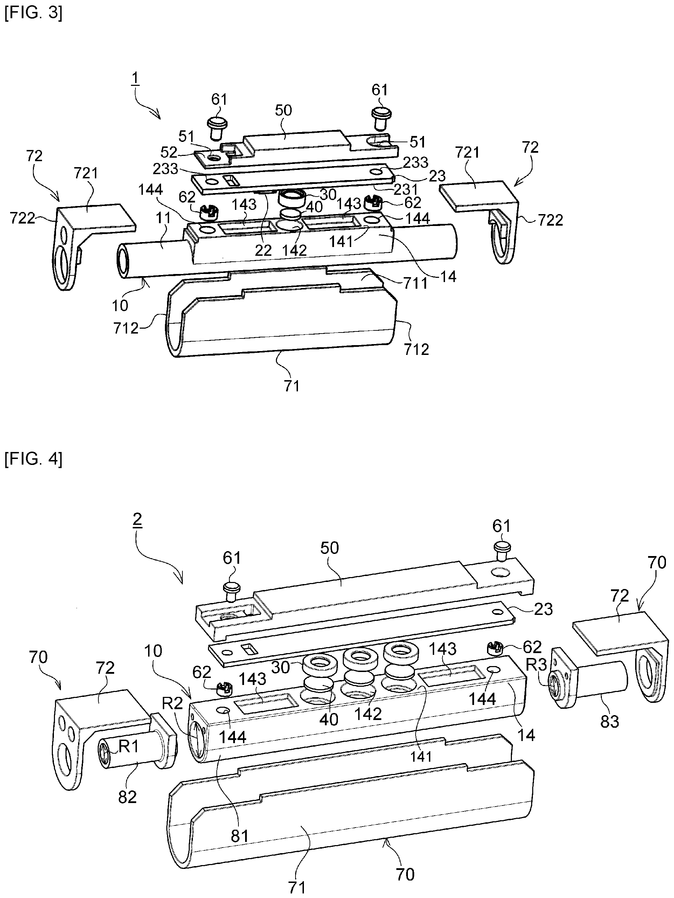

Hereinafter, a fluid sterilization device according to an embodiment of the present invention will be described in detail with reference to the drawings. First, the fluid sterilization device 1 according to the present embodiment will be described with reference to Figs. 1 to 3. Here, Fig. 1 is a vertical cross-sectional view of the fluid sterilization device 1**.** Fig. 2 is an exploded perspective view of the fluid sterilization device 1, viewed from an obliquely lower angle. Furthermore, Fig. 3 is an exploded perspective view of the fluid sterilization device 1, viewed from an obliquely upper angle.

As illustrated in Figs. 1 to 3, the fluid sterilization device 1 according to the present embodiment includes a flow path pipe 10 through which a fluid flows, a light source unit 20, a reflector 30, an ultraviolet light-transmitting window portion (for example, quartz glass) 40, a heat sink 50, metal screws 60, a housing 70, and the like.

Herein, the flow path pipe 10 of the present embodiment is a cylindrical member having a side peripheral wall 11. A flow path 111, through which a fluid to be sterilized (for example, water) flows, is formed inside the side peripheral wall 11. Furthermore, the flow path pipe 10 has a straight tube shape (I-shape), and also has open end portions (an inlet end 12 and an outlet end 13) at opposite ends in the longitudinal direction. Thus, the fluid flows from the inlet end 12 into the flow path pipe 10, flows through the side peripheral wall 11, and flows out from the outlet end 13. However, the form of the flow path pipe 10 is not limited to this. Examples of other forms of the flow path pipe 10 include an L-shaped form.

The flow path pipe 10 of the present embodiment is made of polytetrafluoroethylene (PTFE). However, the material of the flow path pipe 10 is not limited to this. Examples of other materials of the flow path pipe 10 include resins such as a perfluoroethylene propene copolymer (FEP) and perfluoroalkoxyalkane (PFA), and metals such as stainless steel.

Furthermore, as illustrated in Figs. 1 to 3, the flow path pipe 10 of the present embodiment further includes a substrate placement table portion 14 that protrudes radially outward from the side peripheral wall 11 and on which a substrate 23, which will be described later, in the light source unit 20 is placed. As shown in Fig. 3, the substrate placement table portion 14 is an elongated portion that extends along the longitudinal direction of the flow path pipe 10. The substrate placement table portion 14 preferably has a flat substrate placing surface 141. In the present embodiment, the substrate placing surface 141 and the inner surface 231 of the substrate 23 are in contact with each other.

The substrate placement table portion 14 further includes a first through hole 142 that penetrates through to the flow path 111. As shown in Fig. 1, a light emitting element 21 of the light source unit 20 faces the first through hole 142. A reflector 30 and an ultraviolet light-transmitting window portion 40 that surround the light emitting element 21 are disposed in the first through hole 142.

Furthermore, the substrate placement table portion 14 includes a first recess 143 for accommodating electronic components 22 (for example, various electronic components constituting a driving circuit of the light emitting element 21, a connector for connecting the light emitting element 21 and a power source (not shown), and the like), which will be described later, in the light source unit 20. As shown in Figs. 1 to 3, the first recess 143 of the present embodiment includes two recesses arranged side by side in the longitudinal direction of the substrate placement table portion 14. However, the number and the position of the first recess 143 are not limited thereto.

Next, as shown in Figs. 1 to 3, the light source unit 20 includes the light emitting element 21 that emits ultraviolet light (e.g., light having a peak wavelength in 100 nm to 400 nm), the electronic components 22 other than the light emitting element 21 (various electronic components and connectors constituting the driving circuit of the light emitting element 21 described above, etc.), and a (circuit) substrate 23 on which the light emitting element 21 and the electronic components 22 and the like are mounted.

The type of the light emitting element 21 is not particularly limited, and examples thereof include an LED (Light emitting diode) and a semiconductor light emitting element such as a laser diode. The number of the light emitting element 21 of the present embodiment is one, but the present invention is not limited thereto. Furthermore, in the present embodiment, the single light emitting element 21 faces the single first through hole 142, but a plurality of light emitting elements 21 may face the single first through hole **142.**

The substrate 23 of the present embodiment has a flat shape extending in the longitudinal direction of the flow path pipe 10. The light emitting element 21 and the electronic component 22 are mounted on the inner surface 231 of the substrate 23. Furthermore, as described above, the inner surface 231 of the substrate 23 and the substrate placing surface 141 of the substrate placement table portion 14 come into contact with each other. Furthermore, when the substrate 23 is placed on the substrate placement table portion 14, the light emitting element 21 is accommodated in the first through hole 142 and also the electronic component 22 is accommodated in the first recess 143. As a result, when the substrate 23 and the substrate placement table portion 14 are superposed on each other, the substrate placement table portion 14 (the side peripheral wall 11 of the flow path pipe 10) and the light emitting element 21 and the electronic components 22, which are mounted on the substrate 23, can be prevented from physically obstructing each other. As a result, since both of them can be stacked without gaps, the device can be made compact.

Next in the description, the reflector 30 is an annular member that surrounds the light emitting element 21. According to the present embodiment, by providing the reflector 30, ultraviolet light from the light emitting element 21 can be distributed in a manner where the ultraviolet light is emitted to a desired range. As a result, the illuminance of the ultraviolet light in the light distribution range can be increased, so that the sterilization function can be further improved. The ultraviolet light emitted from the light emitting element 21 is reflected (distributed) in a predetermined direction by the reflector 30, passes through the ultraviolet light-transmitting window portion 40, and reaches the flow path 111 of the flow path pipe 10.

Next in the description, the heat sink 50 is in thermal contact with the outer surface 232 of the substrate 23, so that it dissipates heat generated when the light emitting element 21 is driven (when a current is applied). As shown in Fig. 1, the heat sink 50 of the present embodiment extends in the longitudinal direction of the flow path pipe 10 so as to be superposed on the substrate 23. As described above, since the substrate placement table portion 14, the substrate 23, and the heat sink 50 are stacked, a corresponding heat dissipation property is ensured where the heat is dissipated outwards along the radial direction of the flow path pipe 10, and the device is made compact.

Next in the description, the metal screws 60 are used to fix the heat sink 50 and the substrate 23 to the substrate placement table portion 14. The metal screw 60 of the present embodiment includes a fixing screw part 61 and an insert screw part 62. Here, the heat sink 50 is provided with second through holes 51 (see Figs. 2 and 3). Third through holes 233 (see Figs. 2 and 3) are provided in the substrate 23 at positions overlapping with the second through holes 51. Furthermore, as shown in Fig. 1, the fixing screw parts 61 of the present embodiment are inserted into the second through holes 51 and the third through holes 233, respectively.

Furthermore, second recesses 144 are provided at positions overlapping with the second through holes 51 and the third through holes 233 in the substrate placement table portion 14. Furthermore, as shown in Fig. 1, the insert screw parts 62 of the present embodiment are disposed in the second recesses 144. More specifically, the insert screw parts 62 are screwed together with the fixing screw parts 61 that pass through the second through holes 51 and the third through holes 233.

As described above, the heat sink 50 and the substrate 23 are fixed to the substrate placement table portion 14 by the metal fixing screw parts 61, which are inserted into the second through holes 51 of the heat sink 50 and the third through holes 233 of the substrate 23, and by the metal insert screw parts 62, which are disposed in the second recesses 144 of the substrate placement table portion 14. As a result, both the heat sink 50 and the substrate 23 can be more firmly locked.

Furthermore, the fixing screw parts 61 and the insert screw parts 62 are preferably made of a material having high thermal conductivity (for example, various metals such as brass, aluminum, and stainless steel). By having the fixing screw parts 61 and the insert screw parts 62 be made of a material having high thermal conductivity, heat generated when the light emitting element 21 is driven can be dissipated more efficiently.

Although the means of fixing the heat sink 50 and the substrate 23 have been described as the metal screws 60, the aspect of the fixing means is not limited thereto. Examples of other aspects include pin-shaped (rod-shaped) members to be inserted into the second through holes 51, the third through holes 233, and the second recesses 144. In addition, from the viewpoint of appropriately dissipating heat generated during driving of the light emitting element 21, it is preferable that the pin-shaped member or the like be made of a highly thermally conductive material (for example, various metals such as brass, aluminum, and stainless steel). Furthermore, the fluid sterilization device 1 of the present embodiment preferably includes the metal screws 60. Even if the metal screws 60 are removed, the heat sink 50 and the substrate 23 can still be fixed by the housing 70 or the like described later.

Next, the housing 70 is a cylindrical member provided on the radially outer side of the flow path pipe 10 (the side peripheral wall 11). The housing 70 of the present embodiment is made of a resin such as ABS or polypropylene (PP), but is not limited thereto. More specifically, as shown in Figs. 2 and 3, the housing 70 of the present embodiment includes a hollow base portion 71 having an opening 711 on the top side and lid portions 72 configured to cover at least part of the opening 711.

The base portion 71 of the present embodiment has a substantially U-shaped form in a cross-sectional view. However, the form of the base portion 71 is not limited to this. Furthermore, the lid portions 72 of the present embodiment each include a first piece portion 721 that is disposed on the opening 711 side on the top side, and a second piece portion 722 that is substantially orthogonal to the first piece portion 721 and joined to an end edge 712 of the base portion 71.

As shown in Figs. 1 to 3, the flow path pipe 10 is accommodated in the base portion 71 of the housing 70. Furthermore, an outer surface 52 of the heat sink 50 faces the opening 711 of the base portion 71 and comes into contact with (overlaps with) the lid portions 72 (the first piece portions 721). According to the present embodiment, the flow path pipe 10 is accommodated in the base portion 71 of the housing 70, and the outer surface 52 of the heat sink 50 is brought into contact with the lid portions 72 (the first piece portions 721) of the housing 70, thereby preventing detachment of the heat sink 50 and the substrate 23. In addition, the device can be miniaturized.

The embodiment of the present invention has been described in detail above. However, the foregoing description is for the purpose of facilitating understanding of the present invention, and is not intended to limit the present invention. The present invention may include modifications and improvements without departing from the spirit thereof. The invention also includes equivalents thereof.

A modification of the above-described embodiment is exemplified by a fluid sterilization device 2 illustrated in Fig. 4. Here, Fig. 4 is a perspective view of the fluid sterilization device 2 according to the modification, viewed from an upper angle. As shown in Fig. 4, the flow path pipe 10 of the fluid sterilization device 2 according to the modification includes a main pipe portion 81, an inlet pipe portion 82 attached to the inlet end side of the main pipe portion 81, an outlet pipe portion 83 attached to the outlet end side of the main pipe portion 81, and the like.

The inner diameter R2 of the main pipe portion 81 is preferably larger than the inner diameter R1 of the inlet pipe portion 82. With such a structure, the flow rate of the fluid flowing through the flow path 111 can be reduced. As a result, the amount of ultraviolet light irradiation from the light emitting element 21 to the fluid flowing through the main tube portion 81 can be increased. As a result, the sterilization function can be further enhanced. Similarly, the inner diameter R2 of the main pipe portion 81 is preferably made larger than the inner diameter R3 of the outlet pipe portion 83.

### Industrial Applicability

The fluid sterilization device according to the present invention is used for, for example, an ultraviolet light sterilization device, a water cleaner, a hot water supply device, a water supply pipe, a cooling water circulation device, a water server, a drink server, or the like. However, the use thereof is not limited to these. Reference Signs List
1 ... fluid sterilization device
10 ... flow path pipe
   11 ... side peripheral wall
      111 ... flow path
   12 ... inlet end
   13 ... outlet end
   14 ... substrate placement table portion
      141 ... substrate placing surface
      142 ... first through hole
      143 ... first recess
      144 ... second recess
20 ... light source unit
   21 ... light emitting device
   22 ... electronic component
   23 ... substrate
      231 ... inner surface of substrate
      233 ... third through hole
30 ... reflector
40 ... ultraviolet light-transmitting window portion
50 ... heat sink
   51 ... second through hole
   52 ... outer surface of heat sink
60 ... metal screw
   61 ... fixing screw part
   62 ... insert screw part
70 ... housing
   71 ... base portion
      711 ... opening on a top side
   72 ... lid portion
81 ... main pipe portion
82 ... inlet pipe portion
83 ... outlet pipe portion

## Claims

1. A fluid sterilization device comprising:
a cylindrical flow path pipe including a side peripheral wall and a flow path formed inside the side peripheral wall;
a light source unit including a light emitting element, an electronic component other than the light emitting element; and a substrate on which the light emitting element and the electronic component are mounted and that extends in a longitudinal direction of the flow path pipe; and
a heat sink in thermal contact with an outer surface of the substrate, wherein:
the flow path pipe further includes a substrate placement table portion for placing the substrate; and
the substrate placement table portion includes
a first through hole, for accommodating the light emitting element, that penetrates through to the flow path, and
a first recess for accommodating the electronic component.

2. The fluid sterilization device according to claim 1**,** further comprising a metal screw or pin for fixing the heat sink and the substrate to the substrate placement table portion, and
wherein:
a second through hole is provided in the heat sink;
a third through hole is provided at a position overlapping the second through hole in the substrate;
a second recess is provided at a position overlapping the second through hole and the third through hole in the substrate placement table portion; and
the screw or pin is inserted into the second through hole, the third through hole, and the second recess.

3. The fluid sterilization device according to claim 2, wherein the metal screw includes a metal fixing screw part to be inserted into the second through hole and the third through hole, and a metal insert screw part disposed in the second recess and screwed with the fixing screw part.

4. The fluid sterilization device according to claim 1, further comprising a housing, and
wherein:
the housing includes a hollow base portion having an opening at least on a top side, and a lid portion configured to cover at least a part of the opening;
the flow path pipe is accommodated in the base portion; and
an outer surface of the heat sink faces the opening of the base portion to be in contact with the lid portion.

5. The fluid sterilization device according to claim 1, wherein the flow path pipe includes a main pipe portion and an inlet pipe portion having an inner diameter smaller than an inner diameter of the main pipe portion.

6. The fluid sterilization device according to claim 1, wherein the substrate and the flow path pipe are in thermal contact with each other.
